# EUROPEAN PATENT APPLICATION

(11) **EP 4 711 475 A1**
(43) Date of publication of application: **18.03.2026**
(21) Application number: 25201096.2
(22) Date of filing: 09.09.2025
(51) Int. Cl.: C14B 1/00, C14B 17/00, G06Q 10/04, G06Q 10/063, G06Q 50/02, G06Q 50/04, G01N 33/44

(54) **MANAGEMENT SYSTEM FOR A PLANT FOR THE TREATMENT OF HIDES OR SIMILAR**

(30) Priority: 11.09.2024 IT 202400020260
(71) Applicant: Ger Elettronica S.r.l., 36075 Montecchio Maggiore (VI) (IT)
(72) Inventor: BURATO, Bruno, 36075 Montecchio Maggiore (VI) (IT); DAL MONTE, Cesare, 36075 Montecchio Maggiore (VI) (IT); MASETTA, Mirco, 36075 Montecchio Maggiore (VI) (IT)
(74) Representative: Braidotti, Andrea

(57) **Abstract**

Management system (1) for a plant (100) for the treatment of hides or similar, characterised by comprising:
- at least one detection device (40) which is configured to acquire and/or determine data representative of at least one chemical and/or physical characteristic of each hide (12) to be processed or processed in the treatment plant (100),
- at least one reading apparatus (20) which is configured to detect and recognise a code (11) which is applied to each hide (12) to be processed or processed in the treatment plant (100), preferably each reading apparatus (20) being operatively associated with at least one respective detection device (40),
- at least one electronic unit (50) wherein at least one database (51) is loaded,
and wherein:
- said at least one detection device (40) is electronically connected to said at least one electronic unit (50) wherein at least one database (51) is loaded, to thus receive the data acquired and/or determined for each corresponding hide (12) by at least one detection device (40),
- said at least one reading apparatus (20) is electronically connected to said at least one electronic unit (50) wherein at least one database (51) is loaded, to thus receive the code (11) of each hide (12) as recognised by said at least one reading apparatus (20),
- said database (51) is configured and structured so that, within the same database, the code (11) of each hide (12), as recognised by said at least one reading apparatus (20), is matched with the data acquired and/or determined for each corresponding hide (12) by at least one detection device (40) which is operationally associated with said reading apparatus (20).

## Description

### TECHNICAL FIELD

The present invention concerns a management system for a plant for the treatment of hides or similar.

The present invention also relates to a plant for the treatment of hides or similar, which comprises said management system.

Therefore, the present invention finds advantageous use in the technical sector of the treatment of hide or similar, in particular in the processes for transforming raw hides into finished hides.

### STATE OF THE ART

As is known, the process of treating hides to go from raw hide to finished hide envisages a number of intermediate processes on the hide itself, such as fleshing, splitting, tanning, shaving, pressing, drying, buffing, finishing, ironing, etc.

These intermediate processes can be carried out sequentially at the same plant managed by a single entity, or they can be carried out sequentially at geographically distinct locations but always managed by the same entity, or they can be carried out sequentially by different entities at their respective locations.

The hide treatment process lasts several days (on average it can last around 20 days) and for this reason, currently, it is quite complex to follow the batches of hides during the various processes involved in the treatment.

Generally, finished hides are stored or sold according to certain criteria and, in particular, before tanning, semi-finished hides are assigned to a determined type of product that is to be obtained at the end of the process.

A drawback of traditional solutions is the lack of traceability of information relating to processing operations carried out on hides. In more detail, currently, processing operations are not adequately documented, even those performed within the same facility or tannery, nor is it possible to effectively monitor and track hide batches. This compromises the quality of the final product, leading to customer dissatisfaction, financial losses, and increased production costs.

Another drawback of traditional solutions is that hide packaging does not take into account the information accumulated during the treatment process. Packaging is often conducted based solely on data collected during the final stage, such as the hides' quality grade, neglecting data collected during previous processing stages or the specific characteristics of the batches. This makes the process inefficient and heavily dependent on manual operations that inevitably rely on incomplete information.

DE19224304 describes an apparatus for classifying leather pieces including a code reader for detecting the markings on the leather pieces.

DE4012462 shows a leather nesting method wherein leather pieces are arranged on a respective pallet having an identification code.

WO03/038132 describes a hide identification method wherein the hides are pierced.

### OBJECTS OF THE INVENTION

The object of the present invention is to propose a management system for a plant for the treatment of hides or similar, which overcomes - at least in part - the aforementioned drawbacks present in currently known solutions.

Another object of the invention is to track the hides during the subsequent processes envisaged in the hide treatment processing, both for processing operations carried out in the same plant and for processing operations carried out in separate plants, possibly even managed by different entities.

Another object of the invention is to collect information on the hides during the subsequent processing operations involved in the treatment process, both for processing operations carried out in the same plant and for processing operations carried out in separate plants, possibly even managed by different entities.

Another object of the invention is to be able to monitor the hide treatment process in real time.

Another object of the invention is to promptly optimize the hide treatment process.

Another object of the present invention is to propose a system that is highly automated.

Another object of the invention is to propose a system that can be implemented simply, quickly and at low cost.

Another object of the invention is to propose a system that is an improvement and/or alternative to traditional ones.

Another object of the invention is to propose a system with an alternative characterization, both in functional and implementation terms, compared to traditional ones.

### SUMMARY OF THE INVENTION

All these objects - whether considered individually or in any combination thereof - and others that will result from the following description are achieved, according to the invention, with a management system for a plant for the treatment of hides or similar having the characteristics indicated in claim 1 or 3.

The present invention relates to a management system for a plant for the treatment of hides or similar, characterised by comprising:
- at least one detection device that is configured to acquire and/or determine data representative of at least one chemical and/or physical characteristic of each hide to be processed or processed in the treatment plant,
- at least one reading apparatus which is configured to detect and recognise a code which is applied to each hide to be processed or processed in the treatment plant, preferably each reading apparatus being operationally associated with at least one respective detection device,
- at least one electronic unit wherein at least one database is loaded,
and wherein:
- said at least one detection device is electronically connected to said at least one electronic unit wherein at least one database is loaded, to thereby receive the data acquired and/or determined for each corresponding hide by at least one detection device,
- said at least one reading apparatus is electronically connected to said at least one electronic unit wherein at least one database is loaded, to thus receive the code of each hide as recognised by said at least one reading apparatus,
- said database is configured and structured so that, within the same database, the code of each hide, as recognised by said at least one reading apparatus, is matched with the data acquired and/or determined for each corresponding hide by at least one detection device that is operationally associated with said reading apparatus.

Preferably, the system also comprises input means configured to provide said electronic unit with a code for each hide to be processed or processed in the treatment plant, and wherein said database is configured and structured so that, within the same database, the code for each hide (provided by said input means) is matched with the data acquired and/or determined for each corresponding hide by each detection device.

The present invention relates to a management system for a plant for the treatment of hides or similar, characterised by comprising:
- at least one detection device that is configured to acquire and/or determine data representative of at least one chemical and/or physical characteristic of each hide to be processed or processed in the treatment plant,
- input means configured to provide said electronic unit with a code of each hide to be processed or processed in the treatment plant,
- at least one electronic unit wherein at least one database is loaded,
and wherein:
- said at least one detection device is electronically connected to said at least one electronic unit wherein at least one database is loaded, to thereby receive the data acquired and/or determined for each corresponding hide by at least one detection device,
- said database is configured and structured so that, within the same database, the code of each hide is matched with the data acquired and/or determined for each corresponding hide by each detection device.

The present invention also relates to a management system for a plant for the treatment of hides or similar, characterised by comprising:
- at least two detection devices, and wherein:
   - at least one detection device is configured to acquire and/or determine data representative of a respective chemical and/or physical characteristic, of each hide to be processed or processed in the treatment plant, which is different from that detected by the other detection device, and/or
   - at least two detection devices are configured to acquire and/or determine data representative of the same chemical and/or physical characteristic of each hide to be processed or processed in corresponding, distinct from each other, positions of the treatment plant,
   - at least one electronic unit wherein at least one database is loaded,
and wherein:
- said at least two detection devices are electronically connected to said at least one electronic unit wherein at least one database is loaded, to thus receive the corresponding data acquired and/or determined for each corresponding hide by each detection device,
- said database is configured and structured so that, within the same database, a code for each hide to be processed or processed in the treatment plant is matched with the data acquired and/or determined for each corresponding hide by each detection device.

Preferably, the system comprises input means configured to provide said electronic unit with a code for each hide to be processed or processed in the treatment plant, and in said database it is also configured and structured so that, within the same database, the code for each hide, as provided by said input means, is matched with the data acquired and/or determined for each corresponding hide by at least one detection device.

Preferably, the system also comprises at least one reading apparatus that is configured to detect and recognise a code that is applied to each hide to be processed or processed in the treatment plant, preferably each reading apparatus being operatively associated with at least one respective detection device, and wherein:
- said at least one reading apparatus is electronically connected to said at least one electronic unit wherein said at least one database is loaded, to thus receive the code of each hide as recognised by said at least one reading apparatus,
- said database is also configured and structured so that, within the same database, the code of each hide, as recognised by said at least one reading apparatus, is matched with the data acquired and/or determined for each corresponding hide by at least one detection device that is operationally associated with said reading apparatus.

### DESCRIPTION OF THE FIGURES

The present invention is further clarified below in some of its preferred practical embodiments reported for purely exemplifying and non-limiting purposes with reference to the attached drawings, wherein:
- Figure 1: shows a schematic view of a sequence of processing operations in the hide treatment process,
- Figure 2: shows a schematic view of the management system according to the invention for a plant for the treatment of hides or similar,

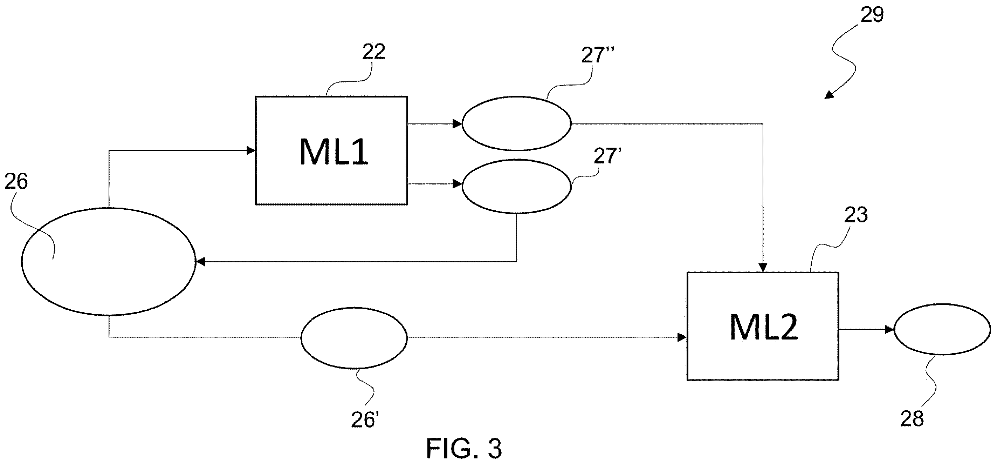

- Figure 3: shows a block diagram of the software loaded and/or executed on the processor of a reading apparatus of the management system according to the invention,
- Figure 4A: shows a side view of a marking apparatus, of the management system according to the invention, for applying a code on a hide,
- Figure 4B: shows a front view of the apparatus of Fig. 4A,
- Figure 5A: shows a side view of a different embodiment of a marking apparatus, of the management system according to the invention, for applying a code on a hide,
- Figure 5B: shows a front view of the apparatus of Fig. 5A,
- Figure 5C: shows a top view of the apparatus of Fig. 5A,

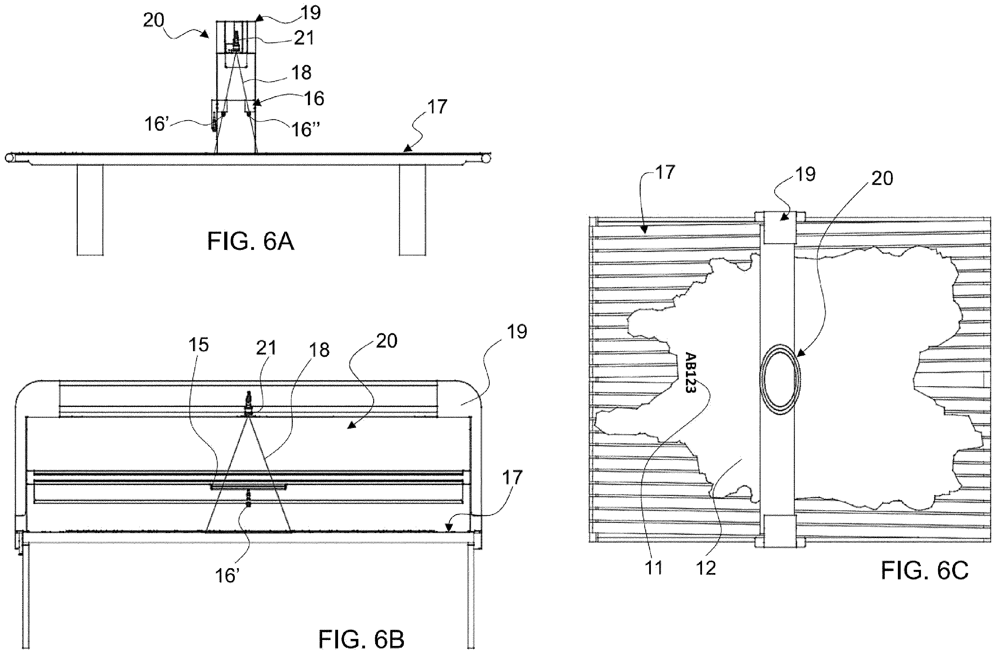

- Figure 6A: shows a side view of a reading apparatus, of the management system according to the invention, for detecting and recognizing a code applied on a hide,
- Figure 6B: shows a front view of the apparatus of Fig. 6A,
- Figure 6C: shows a top view of the apparatus of Fig. 6A,

### DETAILED DESCRIPTION OF THE INVENTION AND OF SOME PREFERRED EMBODIMENTS

The present invention relates to a management system, indicated as a whole by the reference 1, for a plant 100 for the treatment of hide or similar.

Conveniently, the plant 100 for the treatment of hides comprises at least one machine 30 to carry out a processing of the hides and, preferably, comprises two or more machines 30 configured to sequentially perform corresponding, distinct from each other, processing operations on the hides. Conveniently, the plant 100 for the treatment of hides comprises one or more conveyor belts for advancing the hides through the plant itself.

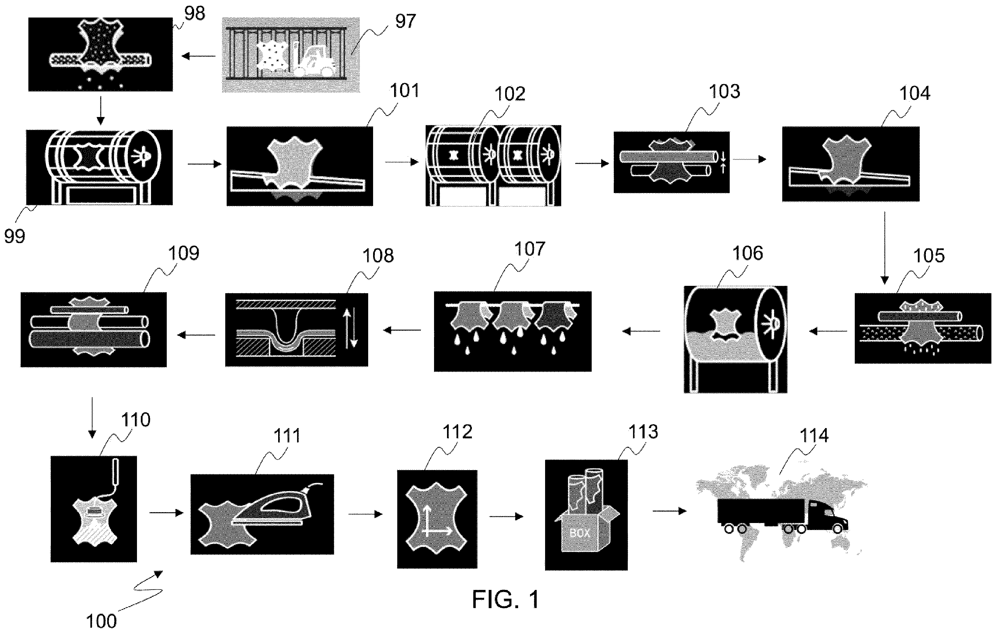

Conveniently, the plant 100 is configured to implement a treatment process of hides or similar. In more detail, as shown in Figure 1, the plant 100 for the treatment of hides may be configured to transform raw hides into finished hides and, to this end, comprises a plurality of processing operations that are performed sequentially on the hides.

In more detail, the processing operations carried out by the machines 30 of the treatment plant 100 include, for example, the following processing operations carried out in sequence starting from the raw hides 97: fleshing 98, alkaline treatment (liming) 99, splitting in the limed state 101, tanning 102, pressing 103, further splitting 104 (so-called *wet blue* hide splitting), shaving 105, re-tanning 106, drying 107, staking 108, buffing 109, finishing 110, ironing 111. Conveniently, the hides thus obtained may then be subjected to measurement 112 and packaging 113 before their shipment 114. Conveniently, each processing carried out in the treatment plant 100 includes a corresponding machine or processing station.

Conveniently, it is intended that the treatment plant 100 may also include only one of the aforementioned processing operations or any subset of the aforementioned processing operations, as well as it may include further traditional hide processing operations (and known to those skilled in the art) which have not been mentioned above.

The management system 1 for the plant 100 for the treatment of hides includes:
- at least one detection device 40 that is configured to acquire and/or determine data representative of at least one chemical and/or physical characteristic of each hide 12 to be processed or processed in the treatment plant 100,
- at least one electronic unit 50 wherein at least one database 51 is loaded
- at least one reading apparatus 20 which is configured to detect and recognise a code 11 which is applied to each hide 12 to be processed or processed in the treatment plant 100, and/or input means configured to provide said electronic unit 50 with a code 11 of each hide 12 to be processed or processed in the treatment plant 100.

Conveniently, the code 11 may be applied to each hide 12 to be processed or processed in the treatment plant 100.

Conveniently, the code 11 applied to each hide 12 may be unique for each hide or may be unique for each batch of hides.

Conveniently, the code 11 applied to each hide 12 may comprise one, two or more alphanumeric characters (possibly with other punctuation, mathematical and/or special characters) and/or other symbols, and/or may comprise a sequence or matrix with dots and/or lines (preferably of a standard type such as barcodes, QR codes, Data Matrix, etc.). Conveniently, the code 11 may be engraved, punched, marked and/or written on each hide 12 to be processed or processed in the treatment plant 100.

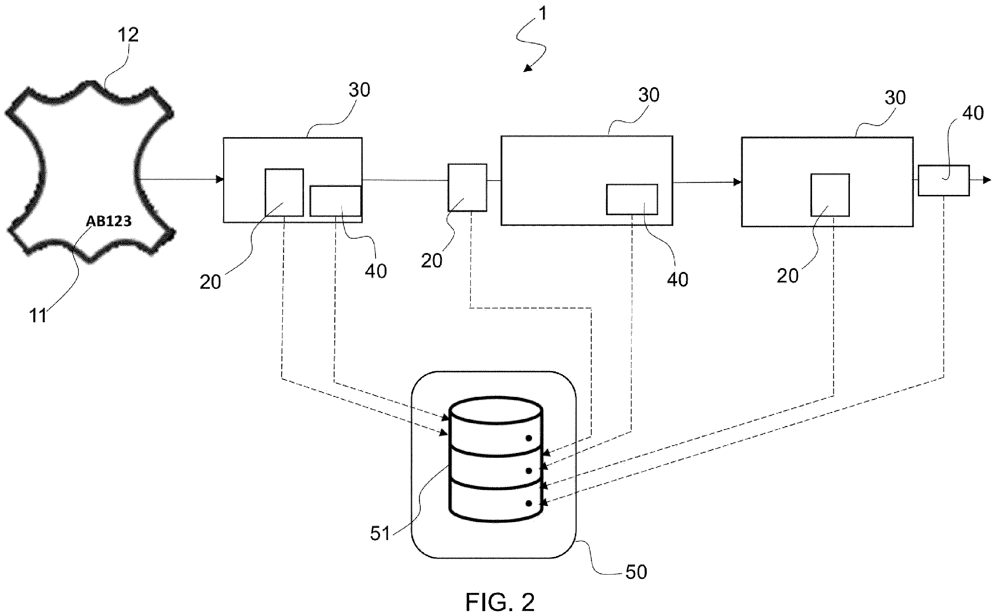

In the embodiment shown in Figure 2, the management system 1 is configured so that a reading apparatus 20 is operatively associated with at least one detection device 40. Preferably, each reading apparatus 20 is operatively associated with a respective detection device 40, or even with two or more respective detection devices 40. Conveniently, each detection device 40 is operatively associated with a reading apparatus 20.

Conveniently, the management system 1 may comprise two or more reading apparatuses 20 which are positioned in sequence within the plant 100 for the treatment of hides.

Preferably, the management system 1 may comprise (at least) one reading apparatus 20 for each machine 30 (or station) provided within the treatment plant 100 for processing the hides. Preferably, each reading apparatus 20 may be installed on board a machine 30 for processing the hides, or may be installed upstream or downstream of said machine 30. Preferably, each reading apparatus 20 is of the stand-alone type. Conveniently, each reading apparatus 20 may be mechanically and electrically independent with respect to a corresponding machine 30 for carrying out a processing of the hides and also with respect to a corresponding detection device 40. Conveniently, the reading apparatus 20 may be mounted on a dedicated conveyor belt or may be mounted on a conveyor belt 17 already provided in the treatment plant 100.

Conveniently, in a possible embodiment, at least one reading apparatus 20 may be installed on board a corresponding detection device 40.

Preferably, the input means configured to provide said electronic unit 50 with the code 11 of each hide 12 to be processed or processed in the treatment plant 100 may comprise:
- an input interface for manual entry of the code by an operator, and/or
- an automatic acquisition/reading interface from physical media (for example by means of an optical reading apparatus of a barcode or equivalent on paper documents or similar), and/or
- an electronic communication interface for receiving the code coming from or transmitted by other external management devices or systems (such as production or logistics information systems).

Conveniently, the input means for providing said electronic unit 50 with the code 11 of each hide 12 to be processed or processed in the plant 100 may be alternative or additional to the reading apparatus 20.

Preferably, the input means for providing said electronic unit 50 with the code 11 of each hide 12 to be processed or processed in the plant 100 may be operationally associated with at least one respective detection device 40.

Preferably, the input means for providing said electronic unit 50 with the code 11 of each hide 12 to be processed or processed in the plant 100 may be provided in multiple (at least two) positions and/or stations along the plant itself, and/or even in multiple positions within the same processing station, for example upstream and downstream of a machine 30.

Advantageously, the management system 1 is configured to operate with hides that are in motion (advancing) within the plant 100. Conveniently, the detection devices 40 are configured to acquire and/or determine data representative of at least one chemical and/or physical characteristic of each hide 12 while in motion and advancing within the plant. Conveniently, the reading apparatus 20 is configured to detect and recognise a code 11 applied to each hide 12 while in motion and advancing within the plant.

Conveniently, each detection device 40 may be configured to detect at least one of the following characteristics of a hide: hide area/surface area, hide thickness, hide color, hide quality (i.e., presence of defects), moisture, and/or softness.

Conveniently, the management system 1 may comprise two or more detection devices 40 which are positioned in sequence with each other within the plant 100 for the treatment of hides.

Conveniently, the management system 1 may comprise two or more detection devices 40, at least one of which is configured to acquire and/or determine data representative of a respective chemical and/or physical characteristic of each hide to be processed or processed in the treatment plant, which is different from that detected by the other detection device. For example, one detection device 40 is configured to detect the area/surface area of the hide, while the other detection device is configured to detect the color of the hide.

Conveniently, the management system 1 may comprise two or more detection devices 40, of which at least two detection devices 40 are configured to acquire and/or determine data representative of the same chemical and/or physical characteristic of each hide 12 to be processed or processed in corresponding, distinct from each other, positions of the treatment plant 100. For example, a detection device 40 configured to detect the color of the hide is provided upstream of a processing machine 30, and another detection device 40 - also configured to detect the color of the hide - is provided downstream of the same processing machine 30.

Conveniently, multiple detection devices 40 can be provided even in the same processing station but in different positions, or even in different positions along the longitudinal extension of a conveyor belt.

Conveniently, the detection device 40 may be a stand-alone device that is subsequently installed on board a machine 30 for carrying out a hide processing, or it may be installed upstream or downstream of said machine 30. Conveniently, the detection device 40 may be incorporated into a machine 30 for carrying out a hide processing.

Conveniently, the detection device 40 may be mechanically and electrically independent of a corresponding machine 30 for carrying out a hide processing. Conveniently, the detection device 40 may be mechanically and/or electrically connected to - and/or incorporated into - a corresponding machine 30 for carrying out a hide processing.

Conveniently, the detection device 40 may be mounted on a dedicated conveyor belt or may be mounted on a conveyor belt already provided in the treatment plant 100.

Said at least one detection device 40 is electronically connected, via wire and/or wirelessly, to said at least one electronic unit 50 wherein at least one database 51 is loaded, to thus receive the data acquired and/or determined for each corresponding hide 12 by at least one detection device 40.

Said at least one reading apparatus 20 is electronically connected, via wire and/or wirelessly, to said at least one electronic unit 50 wherein at least one database 51 is loaded, to thus receive the code 11 of each hide 12 as recognised by said at least one reading apparatus 20.

Conveniently, the input means for providing said electronic unit 50 with a code 11 of each hide 12 are electronically connected, via wire and/or wirelessly, to said at least one electronic unit 50 wherein at least one database 51 is loaded.

Conveniently, the electronic unit 50 may be configured to control and/or command said at least one detection device 40, said at least one reading apparatus 20 and/or said input means for providing said electronic unit 50 with a code 11 of each hide 12.

The electronic unit 50 may be located at/close to the system 100 or may be a remote electronic unit. Conveniently, the electronic unit 50 may comprise at least one processor, such as a real or virtual remote server and/or a cloud system. Preferably, a single electronic unit 50 may be electronically connected to each detection device 40, each reading apparatus 20, and/or the input means for providing said electronic unit 50 with a code 11 of each hide 12.

Conveniently, the database 51 is provided, in a traditional way, with media for storing the data and a processor for processing them (database server), and with software applications (i.e. a database management system) for the efficient creation, manipulation, management and querying of the data stored on the media.

The database 51 is configured and structured so that, within the database itself, the code 11 of each hide 12, as recognised by a reading apparatus 20 and/or provided by said input means, is matched with the data acquired and/or determined for each corresponding hide by at least one detection device 40 which is operationally associated with said reading apparatus 20 and/or said input means.

Conveniently, each record of the database 51 comprises a field that is intended to be filled with the code 11 recognised by a determined reading apparatus 20 and/or provided by input means for a determined hide 12 and also comprises at least one field that is intended to be filled with the data (or based on the data) acquired and/or determined for that determined hide by at least one detection device 40 that is operatively associated with that determined reading apparatus 20 and/or with those determined input means.

In other words, each record of the database 51 comprises the code 11 of a hide 12, as recognised by a reading apparatus 20 and/or provided by the input means, and also the data of the same hide, as acquired and/or determined by a corresponding detection device 40 which is operationally associated with that reading apparatus 20.

Advantageously, in this way, the data acquired and/or determined by the detection device 40 are conveniently linked/referred to a determined hide 12, which is identified - preferably uniquely at hide or batch level - by its code 11 as recognised by the reading apparatus 20 and/or provided by the input means. Advantageously, the chemical/physical characteristics - as determined with or based on the data acquired by each detection device 40 - are correctly associated/matched to the respective hide 12. Therefore, within the plant 100 it is possible to know the characteristics of each hide substantially in real time.

Preferably, at the database level 51, a configuration is envisaged such that each reading apparatus 20 - and/or each input means interface - is pre-associated with the detection device or devices 40 which, in the plant 100, is/are operationally (in particular in terms of position) associated with the respective reading apparatus 20 - and/or with the respective input means interface. For example, within the plant 100, a reading apparatus 20 and/or input means are operationally associated with (at least) one detection device 40 in the sense that they may be physically located more or less close to each other, or they may even be integrated into one another as a single unit, or they may be arranged in such a way that no processing or modification of the hide 12 advancing through the treatment plant 100 occurs between them.

Preferably, the system 1 comprises a software for processing and/or analysis of the data stored in the database 51. Conveniently, said processing and/or analysis software may be loaded and/or executed on the same electronic unit 50 wherein the database 51 is loaded and/or on another electronic unit that interfaces with said database 51. Conveniently, said processing and/or analysis software is configured to perform various analyses, for example statistical analyses of a descriptive and/or inferential type or through automatic learning techniques/algorithms (for example *data mining* or *machine learning* ) for data analysis, using the data stored in the database 51. Conveniently, said processing and/or analysis software is configured to perform quality control analyses (both at the product and manufacturing process(es) level), trend analyses (for example identifying recurring models or patterns for future forecasting or decision-making purposes) and also analyses for process optimization.

Advantageously, the management system 1 may allow for the precise and timely collection of data for each hide 12 in the various processes that, within the treatment plant 100, can be carried out sequentially on each hide 12 in order to transform it from the raw state to the so-called "lime" state (the state of the hide after the hair and residual flesh have been removed) and/or to the so-called "wet" state (the state of the hide after tanning) and/or to the so-called "crust" state (a semi-finished state wherein the hide has undergone further treatments up to a base color that generally occurs in a drum) and/or to the finished state (in which the hide has completed the finishing process, which comprises coloring and aesthetic pressing, and is essentially a finished product). Advantageously, the data thus collected can be used to carry out appropriate analyses.

Advantageously, the management system 1 allows data to be collected even during intermediate processing steps. Therefore, this data can be used or reported on accompanying documents during the final hide packaging phase.

Advantageously, the management system 1 therefore allows for real-time data collection on the processing performed on hides 12 and, if necessary, for prompt optimization of the treatment process. For example, it allows for optimization of upstream processes by adapting them based on the specific characteristics of the hides being processed as detected further downstream. In this way, proactive, precise, and timely action can be taken to ensure maximum effectiveness of the processing performed and improve the quality of the finished product. Advantageously, the management system 1 allows for real-time feedback to be provided to operators, thus permitting rapid intervention in the event of anomalies detected during analysis, and also allows to provide dashboards or visual reports (also including graphs) that facilitate understanding of the analysis results, supporting informed decisions. Advantageously, automatic actions can also be implemented based on the analysis results, such as adjusting machine settings to optimize the corresponding processing without direct operator intervention. Advantageously, based on the analysis results, the processing settings for subsequent batches can be changed and corrected with the aim of refining the entire treatment process.

Furthermore, advantageously, by using a reading apparatus 20 - and/or input means - for each machine 30 for carrying out a processing of the hides within the plant 100 and/or by providing a reading apparatus 20 - and/or input means - for each predefined position along the plant 100, it is possible to track and know substantially in real time the position wherein each hide 12 is located within the plant itself.

### Reading apparatus 20

Preferably, each reading apparatus 20 comprises:
- at least one acquisition device 21 that is configured to acquire digital images of each hide 12 that enters the field of view 18 of said acquisition device 21,
- a processor (not shown) which is electronically connected to said at least one acquisition device 21, to thereby receive the digital images acquired by said device, and wherein a software 29 is loaded which, when executed, is configured to detect and recognise, on the basis of the received images, the code 11 applied on each hide 12.

Preferably, the processor of the reading apparatus 20 may be implemented by means of a computer. Preferably, the reading apparatus 20 may include a user interface (e.g., a display) that is operably associated with the processor, thereby allowing the operator to view the code 11 recognised on each hide 12. Preferably, in one possible embodiment, the user interface of the reading apparatus 20 is also configured to receive as input a code entered by the operator, for example, to modify or correct a code incorrectly recognised by the apparatus.

Preferably, each acquisition device 21 comprises a camera and, more preferably, a linear camera (i.e., a camera that acquisitions an image by acquiring one row of pixels at a time). Conveniently, in the case of a linear camera, the latter and/or the processor of the reading apparatus 20 are configured to assemble the acquired rows of pixels in order to reconstruct a complete image. Conveniently, an encoder (or equivalent) may be provided that sends corresponding signals, used to assemble the rows of pixels in order to reconstruct a complete image, to the acquisition device 21 or to the processor. This is particularly advantageous in order to allow the recognition of the code 11 associated with the hides 12 that are in motion (i.e., while advancing) along the system. Preferably, for each advancement signaled by the encoder, the acquisition device 21 can detect a row of pixels.

Conveniently, each acquisition device 21 is positioned above the hides 12 so as to frame from above the hides as they sequentially transit within the field of view 18 of the corresponding device. Conveniently, each acquisition device 21 is configured and positioned so as to frame within its field of view 18 a predefined part or the entire upper surface of the hides 12 as they sequentially pass through the field of view of the corresponding device.

Conveniently, an acquisition device 21 that is positioned centrally in width (i.e. along a horizontal, or substantially horizontal, direction that is perpendicular to the direction of advancement of the hide) may be provided, but it could also be arranged in an off-center position in width (particularly in the case of multiple acquisition devices 21) with respect to the hide 12 or to a conveyor belt.

Conveniently, in one possible embodiment, each reading apparatus 20 comprises a support structure 19 (e.g., a laterally supported crosspiece) on which at least one acquisition device 21 is mounted. Conveniently, in one possible embodiment, the support structure 19 is configured to support at least one acquisition device 21 above a conveyor belt 17 which sequentially advances the hides through the field of view 18 of the corresponding acquisition device 21.

Preferably, each reading apparatus 20 also comprises illumination means 16 mounted at each acquisition device 21 to illuminate the hide 12 when it enters the field of view 18 of the corresponding acquisition device 21. More preferably, in a possible embodiment, the illumination means 16 comprise two illuminators 16' and 16" which, for example, are positioned respectively upstream and downstream of the acquisition device 21 with respect to the direction of advancement of each hide 12 within the field of view 18 of the acquisition device 21. Conveniently, the illumination means 16 can be configured to emit both visible and non-visible light radiation (at various wavelengths), depending on the needs.

Conveniently, in a possible embodiment, in proximity to the illumination means 16 - and in particular above the latter - a filter 15 may be provided to cover or attenuate the diffusion of the illumination emitted by the means 16, in particular so as to limit the direct and intense diffusion of light which may be annoying or dangerous for the eyes of the operators.

Preferably, the software 29 loaded into the processor and/or executed by the processor of the reading apparatus 20 may be configured to process one or more images 26 of each hide 12 captured by the acquisition device 21 using artificial intelligence and/or automatic learning algorithms *(machine learning).*

The software 29 loaded into the processor and/or executed by the processor of the reading apparatus 20 comprises:
- a first software module 22 provided with a first machine learning model ML1, already trained, which:
   - at the input receives all the images 26 of each hide 12 captured by the acquisition device 21,
   - on output provides an indication 27' of the presence or absence of a code 11 on the image of the hide 12 and also an indication 27" of the position, within the image of the hide 12, where any code 11, if present, is located,
- a second software module 23 configured to implement a second machine learning model ML2, already trained, which:
   - as input receives only selected images 26' of the hide 12 wherein the presence of a code 11 has been detected and also the indication 27"- as provided in output by the first machine learning model ML1 - of the position, within the hide image, where the code 11 is found,
   - as output 28 provides the recognised code 11 in digital format.

Preferably, the indication 27" of the position, within the image of the hide 12, where the code 11 is located includes the coordinates of the vertices of a rectangular (or of another shape) area within which the code 11 is present.

Conveniently, the software 29 is configured so that, among all the images 26 of each hide 12 captured by the acquisition device 21, it selects - based on the indication 27' of the presence or absence of a code 11 on the image of the hide itself - only the images 26' of the hide wherein the presence of a code 11 has been detected. Conveniently, only the images thus selected 26' are sent as input to the second machine learning model ML2. Preferably, the software 29 is configured so as to eliminate the images that have not been so selected, i.e., so as to eliminate the images wherein the presence of a code 11 has not been detected.

Preferably, the software 29 is configured so that among the selected images 26' (i.e. wherein the presence of a code 11 has been detected) relating to the same hide 12, it carries out a further selection based on the characteristics of the images, for example in terms of contrast level and/or other suitably predefined parameters. Conveniently, the selection criterion of the best image is established by considering multiple factors to adapt to the specific purpose of recognizing the code 11. In practice, in a possible embodiment, the software 29 analyses and compares the contrast level (or at least another predefined parameter) of each image 26' containing the code 11 and relating to the same hide 12, to identify at least one image that presents the optimal contrast (or another optimal value of at least a certain predefined parameter) to thus have maximum readability and accuracy in the recognition of the code 11 itself.

Conveniently, the first machine learning model ML1 is pre-trained - and/or can be further trained over time - using a corresponding dataset for supervised learning; conveniently, the corresponding dataset for the first model ML1 comprises an organized collection of records, wherein each record concerns an image of a hide 12 with or without code 11 and comprises - for each image - what is sent as input to the corresponding ML1 model and also what provided on output by the corresponding model ML1.

Conveniently, the second machine learning model ML2 is pre-trained and/or can be further trained using a corresponding dataset for supervised learning; conveniently, the corresponding dataset for the second model ML2 comprises an organized collection of records, wherein each record concerns an image of a hide 12 with a code of 11 and includes - for each image - what sent as input to the corresponding model ML2 and also what provided on output by the corresponding model ML2.

### Marking device 60

Preferably, the management system 1 also comprises a marking apparatus 60 for applying a code 11 to a hide 12 to be processed or processed, or to a batch of hides to be processed or processed, in the treatment plant 100. Conveniently, the code 11 applied to each hide 12 by the marking apparatus 60 may be unique for each hide or may be unique for each batch of hides.

Preferably, it is the management system 1 itself which may comprise a marking apparatus 60, however in other possible embodiments, the marking apparatus 60 for applying the code 11 on each hide 12 may be external to the management system 1 or may be operationally and/or physically independent with respect to the management system 1.

Conveniently, the code 11 to be applied by the marking apparatus 60 may be set by the operator by acting on a control interface of the apparatus 60, or it may be received from external systems.

Preferably, the marking apparatus 60 is configured to apply the code 11 to the hide 12 by laser radiation. Conveniently, in other possible embodiments, the marking apparatus 60 can be configured to apply the code 11 to the hide 12 through mechanical punching or drilling. Conveniently, in other possible embodiments, the marking apparatus 60 can be configured to apply the code 11 to the hide 12 by inkjet (or equivalent substance) or by thermal transfer.

Conveniently, the code 11 applied by the marking apparatus 60, through laser beam or through mechanical punching/drilling, may be obtained so as to pass through the entire thickness of the hide 12 or by means of an incision that only partially penetrates the thickness of the hide itself.

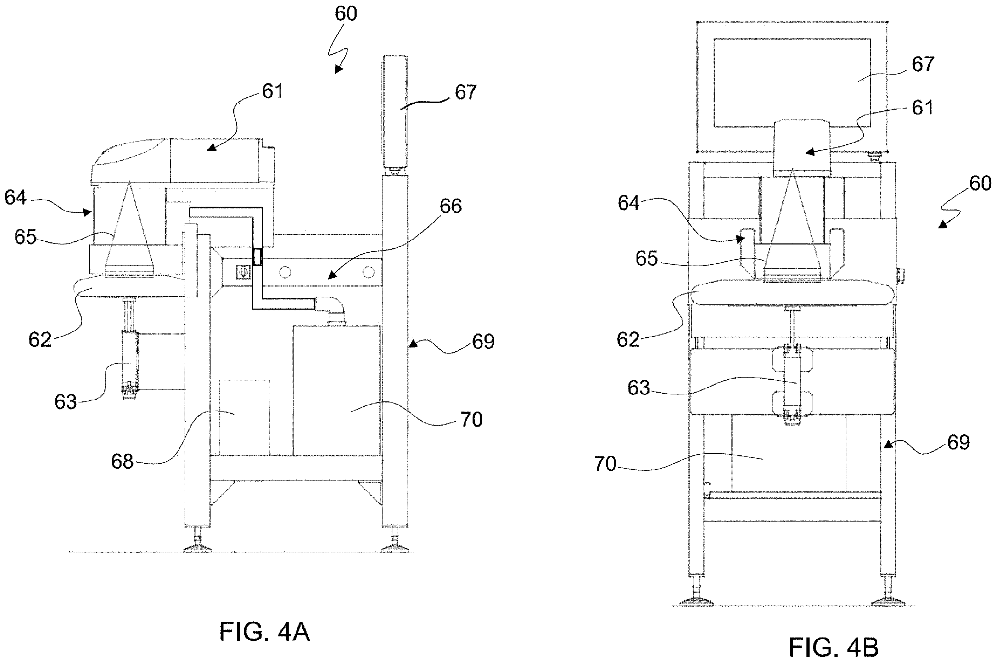

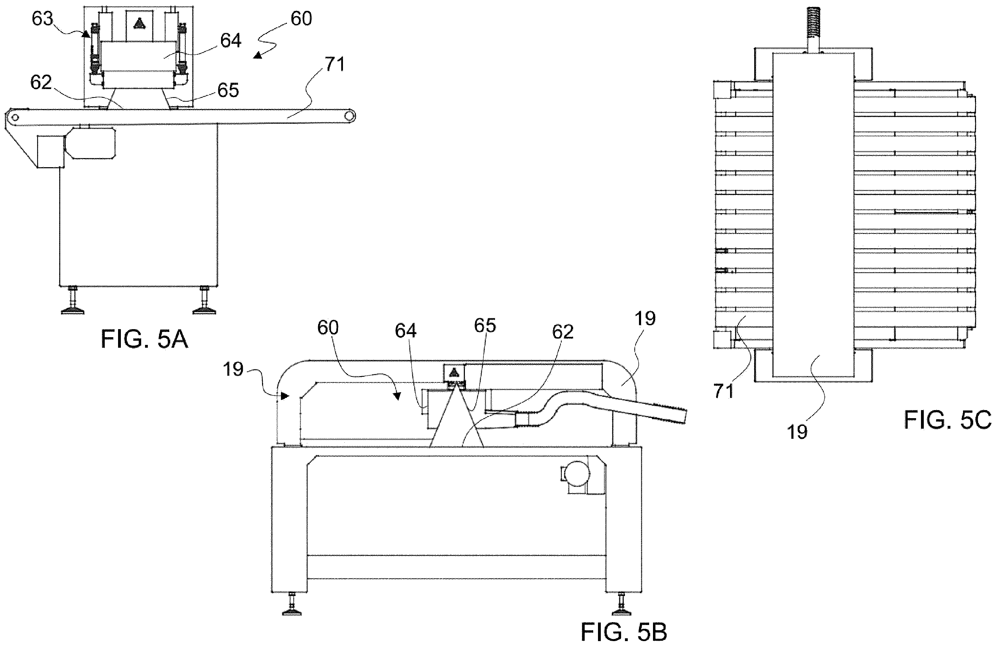

Conveniently, the marking apparatus 60 can be mechanically/structurally of the stand-alone type (see Figs. 4A and 4B) or it can be installed on board a machine 30 for carrying out a hide processing or at a dedicated conveyor belt 71 or one already envisaged in the plant 100 (see Figs. 5A, 5B and 5C).

The marking apparatus 60 may comprise a corresponding support structure 69, which may be a dedicated support structure (see Figs. 4A and 4B) or which may be mounted above a conveyor belt 71 already provided in the plant 100 (see Figs. 5A, 5B and 5C).

In a preferred embodiment, the marking apparatus 60 comprises at least one laser device 61 which is configured to apply the code 11 onto each hide 12 by laser engraving (which - depending on the settings - can completely or partially penetrate the thickness of the hide).

Preferably, in one possible embodiment, the laser device 61 is fixed (i.e. it is not movable in any direction ). In another possible embodiment, the laser device 61 can be mounted on a movable structure, for example along one or more mutually perpendicular axes, to ensure flexibility in the marking coordinates and allow the processing of hides 12 with variable shapes and dimensions.

Preferably, the laser device 61 has a field of action (for example 170 x 170 or 300 x 300) such that it can simultaneously apply a code 11 of several characters to each hide 12.

The marking apparatus 60 comprises a base 62 for receiving the hide 12 to be marked and, conveniently, such base 62 can be defined by a dedicated shelf (see Figs. 4A and 4B) or by the upper surface of a conveyor belt 71 (see Figs. 5A, 5B and 5C).

The marking apparatus 60 comprises a casing 64 for internally defining a working chamber, within which the field of action 65 of the laser device 61 is located. Conveniently, the working chamber defined by the casing 64 is fluidly connected to a suction device 70, thus allowing the suction of gases (and in particular fumes) from the working chamber. Conveniently, the casing 64 may substantially define a suction hood.

Preferably, in one possible embodiment, the marking apparatus 60 may comprise cooling means 68 (e.g. a *chiller*) for the laser device 61.

Preferably, the marking apparatus 60 comprises movement means 63 (for example, one or more actuators) for moving the base 62 receiving the hide 12 closer to/further away from the casing 64, or vice versa. Advantageously, for safety reasons, during the action of the laser device 61, the lower edge of the casing 64 is substantially in contact, or in close proximity, with the base 62 receiving the hide to be treated; in particular, this allows for the suction of the fumes created by laser engraving of the hide 12 and also protects operators from the potential dangers of the laser beam, including risks to the eyes and possible cuts.

Conveniently, in a possible embodiment (see Figs. 4A and 4B), the movement means 63 are configured to move the base 62 so as to bring the base itself, on which the hide 12 to be marked is positioned, substantially into contact, or in close proximity, with the lower edge of the casing 64. In particular, in this case, the movement means 63 move the base 62 receiving the hide 12 to be marked closer to/further away from the laser device 61, thus allowing the positioning and removal of the hide to be marked.

Conveniently, in a possible embodiment (see Figs. 5A, 5B and 5C), the movement means 63 are configured to move the casing 64 towards/away from the base 62 (which can be defined by the upper shelf of the conveyor belt 71) on which the hide to be marked is positioned.

Conveniently, in one possible embodiment, the marking apparatus 60 may comprise a control interface 66 and a display 67 (e.g. a monitor).

Conveniently, the marking apparatus 60 may apply the code 11 to the hide 12 while it is substantially stationary within the field of action 65 of the laser device 61 or to the moving hide (which is advanced by a conveyor belt 71) as it passes through the field of action 65 of the laser device 61.

The present invention also relates to a plant 100 for the treatment of hides for sequentially performing one or more processing operations on the hides, preferably for sequentially performing a plurality of processing operations to transform the hides 12 from the raw state to a finished state. The plant 100 comprises one or more machines 30, arranged in sequence, for performing a processing operation on the hides and at least one conveyor belt for advancing the hides 12 through the system itself. Conveniently, at least two machines 30 are configured to sequentially perform corresponding, different from each other, processing operations on the hides. The plant 100 comprises a management system 1 as described herein.

From what has been said it is clear that the solution according to the invention is significantly advantageous compared to known solutions and, in particular, allows the above-mentioned purposes to be achieved.

The present invention has been illustrated and described in some of its preferred embodiments, but it is understood that executive variations may be made to them in practice, without however departing from the scope of protection of the present patent for industrial invention.

## Claims

1. Management system (1) for a plant (100) for the treatment of hides or similar, **characterised by** comprising:
- at least one detection device (40) which is configured to acquire and/or determine data representative of at least one chemical and/or physical characteristic of each hide (12) to be processed or processed in the treatment plant (100),
- at least one reading apparatus (20) which is configured to detect and recognise a code (11) which is applied to each hide (12) to be processed or processed in the treatment plant (100), preferably each reading apparatus (20) being operatively associated with at least one respective detection device (40),
- at least one electronic unit (50) wherein at least one database (51) is loaded,
and wherein:
- said at least one detection device (40) is electronically connected to said at least one electronic unit (50) wherein at least one database (51) is loaded, to thus receive the data acquired and/or determined for each corresponding hide (12) by at least one detection device (40),
- said at least one reading apparatus (20) is electronically connected to said at least one electronic unit (50) wherein at least one database (51) is loaded, to thus receive the code (11) of each hide (12) as recognised by said at least one reading apparatus (20),
- said database (51) is configured and structured so that, within the same database, the code (11) of each hide (12), as recognised by said at least one reading apparatus (20), is matched with the data acquired and/or determined for each corresponding hide (12) by at least one detection device (40) which is operationally associated with said reading apparatus (20).

2. System according to claim 1, comprising input means for providing said electronic unit (50) with a code (11) of each hide (12) to be processed or processed in the treatment plant (100), and wherein said database (51) is configured and structured so that, within the same database, the code (11) of each hide, which is provided by said input means, is matched with the data acquired and/or determined for each corresponding hide (12) by each detection device (40).

3. Management system (1) for a plant (100) for the treatment of hide or similar, **characterised by** comprising:
- at least one detection device (40) which is configured to acquire and/or determine data representative of at least one chemical and/or physical characteristic of each hide (12) to be processed or processed in the treatment plant (100),
- at least one electronic unit (50) wherein at least one database (51) is loaded,
- input means for providing said electronic unit (50) with a code (11) of each hide (12) to be processed or processed in the treatment plant (100),
and wherein:
- said at least one detection device (40) is electronically connected to said at least one electronic unit (50) wherein at least one database (51) is loaded, to thus receive the data acquired and/or determined for each corresponding hide (12) by at least one detection device (40),
- said database (51) is configured and structured so that, within the same database, the code (11) of each hide is matched with the data acquired and/or determined for each corresponding hide (12) by each detection device (40).

4. System according to one or more of the preceding claims, comprising at least two detection devices (40), of which at least one detection device (40) is configured to acquire and/or determine data representative of a respective chemical and/or physical characteristic, of each hide (12) to be processed or processed in the treatment plant (100), which is different from that detected by the other detection device (40).

5. System according to one or more of the preceding claims, comprising at least two detection devices (40) which are configured to acquire and/or determine data representative of the same chemical and/or physical characteristic of each hide (12) to be processed or processed in corresponding, distinct from each other, positions of the treatment plant (100).

6. System according to one or more of the preceding claims, wherein said system (1) comprises a software for processing and/or analysis of the data stored in the database (51), which is preferably configured to perform statistical analyses of a descriptive and/or inferential type or to implement automatic learning algorithms for data analysis, more preferably to thereby identify recurring models or patterns for future forecasting or decision-making purposes and/or for quality control analysis and/or for process optimization.

7. System according to one or more of the preceding claims, wherein it is envisaged:
- a reading apparatus (20), and/or input means for providing said electronic unit (50) with a code (11) of each hide (12), for each machine (30) for carrying out a processing of the hides (12) envisaged within the plant (100), and/or
- a reading apparatus (20), and/or input means for providing said electronic unit (50) with a code (11) of each hide (12), for each predefined position along the plant (100).

8. System according to one or more of the preceding claims, wherein each reading apparatus (20) comprises:
- at least one acquisition device (21) which is configured to acquire digital images of each hide (12),
- a processor which is electronically connected with said at least one acquisition device (21), to thereby receive the digital images acquired by said device (21), and wherein a software (29) is loaded which, when executed, is configured to detect and recognise, on the basis of the received images, the code (11) applied on each hide (12).

9. System according to the preceding claim, wherein each acquisition device (21) comprises a linear camera and illumination means (16) mounted at each acquisition device (21) to illuminate the hide (12) when it enters the field of view (18) of the corresponding acquisition device (21).

10. System according to claims 8 or 9, wherein each acquisition device (21) is positioned above the hides (12) so as to frame from above the hides which sequentially transit within the field of view (18) of the corresponding device (21), and wherein each acquisition device (21) comprises:
- a linear camera that captures an image by acquiring at least one row of pixels at a time,
- an encoder that sends corresponding signals to the acquisition device (21) or to the processor used to assemble the rows of pixels acquired by the linear camera in order to reconstruct a complete image.

11. System according to one or more of the preceding claims, wherein the software (29) loaded into the processor and/or executed by the processor of the reading apparatus (20) comprises:
- a first software module (22) provided with a first machine learning model (ML1), already trained, which:
- as input receives all the images (26) of each hide (12) captured by the acquisition device (21),
- on output provides an indication (27") of the presence or absence of a code (11) on the image of the hide (12) and also an indication (27") of the position, within the image (26) of the hide (12), where the code (11), if present, is located,
- a second software module (23) configured to implement a second machine learning model (ML2), already trained, which:
- as input receives only selected images (26') of the hide (12) wherein it has been detected the presence of a code (11) and also the indication (27"), as provided on output by the first machine learning model ML1, of the position where the code (11) is located within each selected image (26') of the hide (12),
- as output (28) provides the code (11) recognised in digital format.

12. System according to one or more of the preceding claims, wherein said system (1) also comprises a marking apparatus (60) for applying a code (11) on a hide (12) to be processed or processed in the treatment plant (100).

13. System according to the preceding claim, wherein the marking apparatus (60) comprises at least one laser device (61) which is configured to apply the code (11) on each hide (12) by laser engraving.

14. System according to the preceding claim, wherein the marking apparatus (60) comprises:
- a base (62) for receiving the hide (12) to be marked,
- a housing (64) for internally defining a working chamber, within which the field of action (65) of the laser device (61) is located, and wherein preferably the working chamber is fluidly connected to a suction device (70),
- movement means (63) to move the base (62) receiving the hide (12) closer to/further away from the casing (64), or vice versa.

15. Plant (100) for the treatment of hides to sequentially carry out one or more processing operations on the hides (12), said plant (100) comprising one or more machines (30) in sequence for processing hides (12) and at least one conveyor belt for advancing the hides (12) through the plant (100), and **characterised by** also comprising a management system (1) of the hides (12) according to one or more of the preceding claims.
